# EUROPEAN PATENT SPECIFICATION

(11) **EP 2 550 926 B1**
(45) Date of publication and mention of the grant of the patent: **27.09.2017**
(21) Application number: 10848466.8
(22) Date of filing: 25.10.2010
(51) Int. Cl.: A61B 1/00, A61B 34/30, A61B 34/37

(54) **MEDICAL MANIPULATOR SYSTEM**
MEDIZINISCHES MANIPULATORSYSTEM
SYSTÈME DE MANIPULATION MÉDICALE

(30) Priority: 23.03.2010 JP 2010067000
(43) Date of publication of application: 30.01.2013
(73) Proprietor: Olympus Corporation, Tokyo 192-8507 (JP)
(72) Inventor: NAMIKI, Hirotaka, Hachioji-shi, Tokyo 192-8507 (JP)
(74) Representative: Schicker, Silvia
(86) International application number: PCT/JP2010/068875
(87) International publication number: WO 2011/118074

(56) References cited:
- JP-A- 9 066 056
- JP-A- 9 066 056
- JP-A- 2004 105 539
- JP-A- 2004 105 539
- JP-A- 2009 525 098
- JP-A- 2009 525 098
- US-A- 5 784 542
- US-B2- 7 155 316

## Description

### Technical Field

The present invention relates to a medical manipulator system for performing diagnosis, treatment, and the like by manipulating manipulators inserted in, for example, a living body.

### Background Art

Conventionally, there has been proposed a medical treatment technique which uses a robot to achieve manpower saving in medical facilities. For example, in the field of surgery, there has been proposed a surgical robot system which precisely performs artificial hip joint replacement for a patient by using a multi-degree-of-freedom manipulator. In addition, to accurately obtain position information in the body cavity of a patient, a robot system which performs endoscopic surgical operation has been proposed.

Recently, an endoscopic surgical operation has become popular, in which the operator forms an insertion hole in the body wall such as an abdominal cavity and percutaneously inserts an endoscope or surgical instrument into the body cavity through the insertion hole, thereby performing various kinds of treatments in the body cavity.

Such an operative procedure is a less invasive procedure which does not require a large incision, and is widely used for cholecystectomy, the operation of extracting part of the lung, or the like. In order to improve the operability in such an operative procedure, a medical manipulator based on a master-slave scheme has been devised.

A medical manipulator system using a plurality of medical manipulators is used for a great variety of complicated surgical operations. In a surgical operation using a plurality of medical manipulators, some measures need to be taken to prevent, for example, some of the manipulators which have performed some kind of operation which is not intended by the operator from going out of control and inflicting an excessive force on the operator or the patient.

That is, when using a medical manipulator system, the operator needs to quickly and accurately decide which manipulator controller he/she should shut down and to take extreme measures in case of an emergency.

Under the circumstances, demands have arisen for a medical manipulator system which can quickly take measures against operation failures which have occurred in manipulators in a surgical operation using the manipulators. As such a technique, for example, Japanese Patent No. 3717552 has proposed the following technique. That is, Japanese Patent No. 3717552 discloses a forcible shutdown means for providing measures against operation failures (for example, contact/collision between medical manipulators) which occur in some of a plurality of medical manipulators in a surgical operation using the manipulators.

More specifically, Japanese Patent No. 3717552 discloses an arrangement in which the above forcible shutdown means is implemented by a foot pedal and is provided for each manipulator. In addition, the operator of the medical manipulator system operates these forcible shutdown means. In such an arrangement, a person other than the operator, for example, an assistant on the slave side (patient side) of the manipulator system of the master-slave scheme, cannot perform emergency shutdown of a manipulator according to his/her own judgment. This therefore poses a problem in terms of operability in case of an emergency.

US 5,784,542 discloses a robot system with a master robot and a slave robot. A control panel is provided which provides remote access to switches and indications needed to operate the robot system. The control panel can have a PANIC STOP switch to halt operation quickly, in particular to freeze motion of the slave robot if an error is observed.

### Disclosure of Invention

The present invention has been made in consideration of the above situation, and has as its object to provide a medical manipulator system with improved operability in case of an emergency.

The above object is achieved with a medical manipulator system including the features of claim 1.

Advantages of the invention will be set forth in the description which follows, and in part will be obvious from the description, or may be learned by practice of the invention. The advantages of the invention may be realized and obtained by means of the instrumentalities and combinations particularly pointed out hereinafter.

### Brief Description of Drawings

FIG. 1 is a view showing an example of the arrangement of a medical manipulator system according to an embodiment of the present invention.
FIG. 2 is a schematic view showing an actual image of the medical manipulator system.
FIG. 3 is a view showing the layout of equipment in an operation room when the medical manipulator system is applied to an intraperitoneal surgery.
FIG. 4 is a view schematically showing the arrangement of the actuation mechanism of a surgical instrument of the medical manipulator system.
FIG. 5 is a view schematically showing the arrangement of the actuation mechanism of an endoscope.
FIG. 6 is a flowchart showing processing unique to the medical manipulator system according to an embodiment of the present invention.
FIG. 7 is a view showing an example of the arrangement of a medical manipulator system according to a modification of an embodiment of the present invention.
FIG. 8 is a schematic view showing an actual image of the medical manipulator system according the modification of the embodiment.

### Best Mode for Carrying Out the Invention

An embodiment of the present invention will be described below with reference to the accompanying drawing.

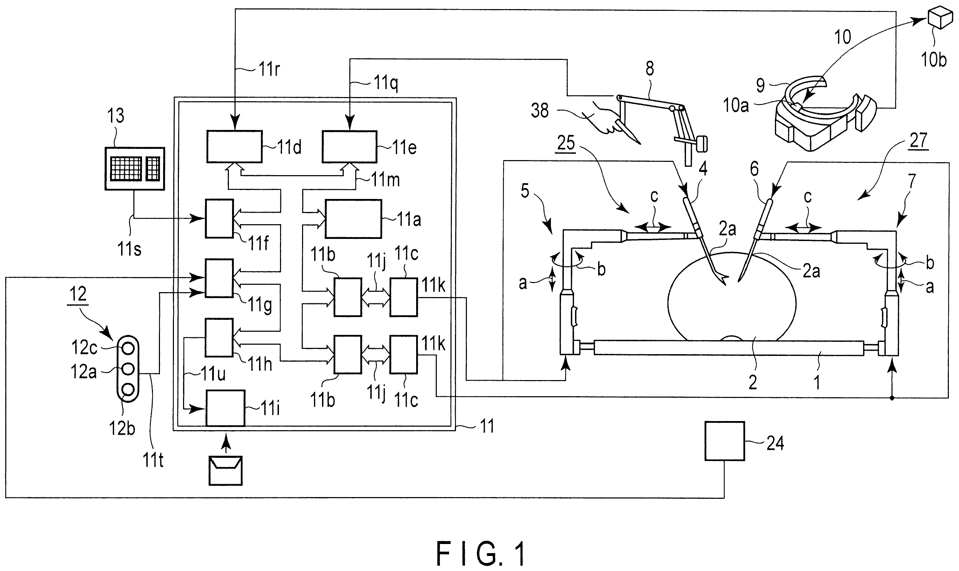

FIG. 1 is a view showing an example of the arrangement of a medical manipulator system according to an embodiment. As shown in FIG. 1, the medical manipulator system includes a controller 11, a switch 12, a keyboard 13, an operating table 1 on which a patient 2 is placed, a treatment slave manipulator 25 constituted by a surgical instrument 4 and a treatment arm 5, an observation slave manipulator 27 constituted by an endoscope 6 and an observation arm 7, a master arm 8 operated by an operator (surgeon) 38, a head-mounted display (HMD) 9, a magnetic sensor 10 constituted by a magnetic sensor sensing unit 10a and a magnetic sensor source unit 10b, a switch 12 constituted by an operation switch 12a disposed near the operator 38 (within an operable range), an emergency shutdown switch 12b, and a shutdown cancellation switch 12c, and a slave-side emergency shutdown switch 24 disposed on the slave side.

Note that the respective members in FIG. 1 have the positional relationship shown in FIG. 1 because of the space limitation and differ from that in an actual arrangement.

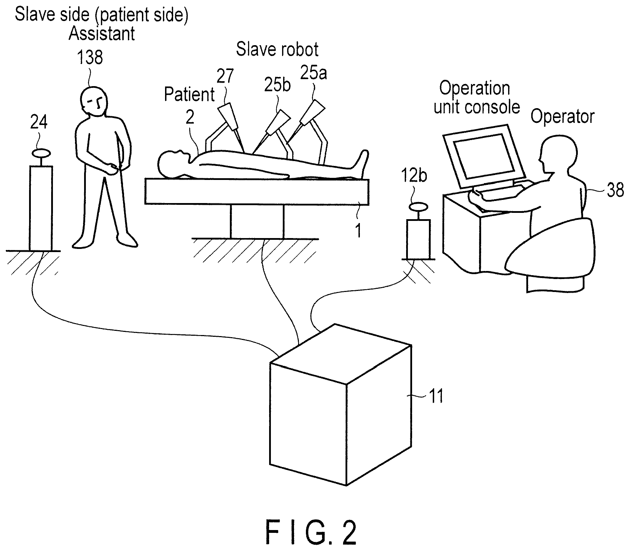

FIG. 2 shows an image illustrating the schematic arrangement of the medical manipulator system according to this embodiment. On the slave side (patient side), the patient 2 is placed on the operating table 1, and the slave-side emergency shutdown switch 24 is disposed near an assistant 138. On the master side (operator side), the switch 12 is disposed near the operator 38.

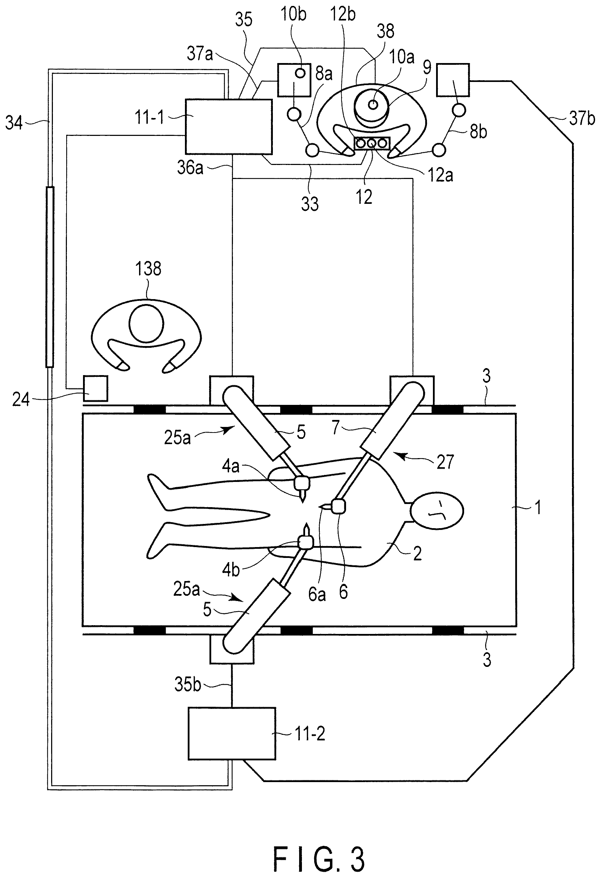

FIG. 3 shows the layout of equipment in an operation room when the above medical manipulator system is applied to an intraperitoneal surgery. Referring to FIG. 3, for the sake of a description of control closer to a real situation, assume the use of two treatment slave manipulators 25 (treatment slave manipulators 25a and 25b), a controller 11-1 as the controller 11 which controls the operation of treatment slave manipulator 25a and observation slave manipulator 27, and a controller 11-2 as the controller 11 which controls the operation of treatment manipulator 25b (although some system arrangement uses three or more slave manipulators, the following will exemplify the above system).

Referring to FIG. 3, the assistant 138 is near the operating table 1 in addition to the equipment (for example, the master arm 8) on the maser side and the operator 38 who manipulates it. The emergency shutdown switch 24 is disposed near the operating table 1. Equipment on the maser side may be disposed in an area remote from the operating table 1 or in another room.

The arrangement and function of the medical manipulator system according to this embodiment will be described below with reference to FIGS. 1, 2, and 3.

The operating table 1 is the one for observation/treatment of the patient 2. Bedside rails 3 are provided on the two sides of the operating table 1. The treatment arm 5 and the observation arm 7 for positioning the surgical instrument 4 and the endoscope 6 in the body cavity of the patient are detachably mounted on the bedside rails 3.

Note that the surgical instrument 4 and the endoscope 6 are inserted in the body cavity of the patient 2 through an insertion hole 2a formed in his or her body wall.

Free joint mechanisms 19 which are joint portions each having a plurality of degrees of freedom connect the treatment arm 5 to the surgical instrument 4 and connect the observation arm 7 to the endoscope 6. This mechanism prevents excessive force from acting on the insertion hole 2a even if the patient 2 moves during a surgical operation and the position of the insertion hole 2a shifts.

The treatment arm 5 and the observation arm 7 are configured to mechanically perform vertical extending/contracting operation (in the direction indicated by an arrow a in FIG. 2), rotating operation (in the direction indicated by an arrow b in FIG. 2), and horizontal extending/contracting operation (in the direction indicated by an arrow c in FIG. 2). In order to realize such movements, actuators (not shown) are disposed in the respective arms. Note that as these actuators, for example, servo motors used for the positioning of a robot are used.

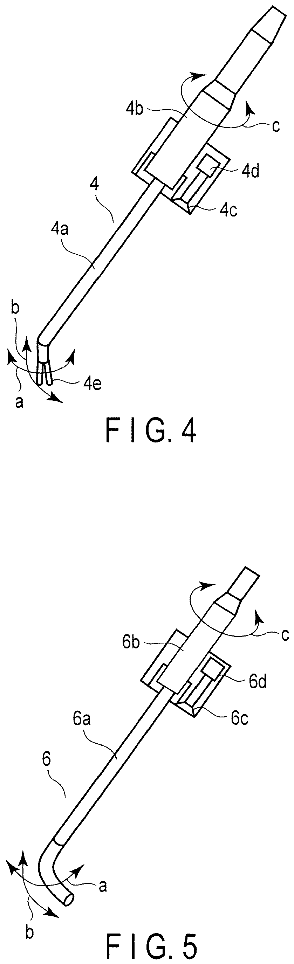

FIG. 4 is a view schematically showing the actuation mechanism of the surgical instrument 4 of the manipulator of the medical manipulator system according to this embodiment. FIG. 5 is a view schematically showing the actuation mechanism of the endoscope 6.

An insertion portion 4a of the surgical instrument 4 mounted on the distal end of the treatment arm 5 has a distal end portion which is configured to bend in the directions indicated by arrows a and b in FIG. 4. Such bending operation is performed by pulling a wire (not shown) extending through the insertion portion 4a by driving a servo motor with a decelerator (not shown) provided in a servo motor accommodation portion 4b of the surgical instrument 4.

The surgical instrument 4 is configured to rotate in the direction indicated by an arrow c in FIG. 4. Such rotating operation is performed by actuating a rotating mechanism (not shown) by driving a servo motor 4d provided in a free joint arm joint portion 4c. A distal end forceps portion 4e of the surgical instrument 4 is, in particular, provided with an opening/closing mechanism for opening/closing the distal end forceps portion 4e. This opening/closing mechanism is actuated by pushing and pulling a rod or wire member extending through the insertion portion 4a by driving the servo motor (not shown) provided in the servo motor accommodation portion 4b.

Likewise, an insertion portion 6a of the endoscope 6 which is mounted on the distal end of the observation arm 7 is configured to bend in the directions indicated by arrows a and b in FIG. 5. Such bending operation is performed by pulling a wire (not shown) extending through the insertion portion 6a by driving the servo motor (not shown) provided in a servo motor accommodation portion 6b of the endoscope 6.

The endoscope 6 is also configured to rotate in the direction indicated by an arrow c in FIG. 5. This rotating operation is performed by actuating a rotating mechanism (not shown) by driving a servo motor 6d provided in a free joint arm joint portion 6c.

As described above, treatment slave manipulators 25a and 25b are obtained by combining the surgical instruments 4 and the treatment arms 5, and the observation slave manipulator 27 is obtained by combining the endoscope 6 and the observation arm 7.

The master arm 8 is an input means for treatment slave manipulators 25a and 25b. Likewise, the HMD 9 is an input means for the observation slave manipulator 27. The master arm 8 is constituted by a plurality of link mechanisms. The respective links constituting the link mechanisms are provided with encoders (not shown) for position detection. Detecting the operation of each link by using this encoder can detect the moving amount of the master arm 8.

An electromagnetic clutch (not shown) is mounted on each arm link of the master arm 8 to prevent the master arm 8 from moving under its own weight when the operator 38 releases the master arm 8.

That is, this electromagnetic clutch restricts the movement of the master arm 8 so as to prevent it from moving unless when it is necessary. In addition, when actually moving treatment slave manipulators 25a and 25b in the master-slave mode, the operator controls the actuation of the electromagnetic clutches by operating the operation switch 12a. That is, the operation switch 12a allows to lock and unlock the operation of the master arm 8.

Alternatively, as a clutch function for slave manipulator driving operation, it is possible to lock/unlock the operation of treatment slave manipulators 25a and 25b by using the operation switch 12a. This can be implemented by, for example, controlling outputs to the servo motors in accordance with the state of the operation switch 12a.

The master-slave mode is a mode of allowing to transfer the movement of the master arm 8 as an input means to treatment slave manipulators 25a and 25b, i.e., a mode of allowing treatment slave manipulators 25a and 25b to follow the movement of the master arm 8 (this also applies to the relationship between the HMD 9 and the observation slave manipulator 27 (which will be described later)).

The HMD 9 includes a display (not shown) which displays a picture observed by the endoscope 6. This display is provided to be located at a position corresponding to the eyes of the surgeon when the operator wears the HMD 9 on his/her head. In addition, the HMD 9 is configured to allow the operator to always observe the picture captured at the distal end of the endoscope 6 with the above display, no matter how his/her head moves. The HMD 9 with this arrangement saves the trouble on the part of the surgeon of performing cumbersome operation, for example, shifting his/her line of sight to the TV monitor installed in an operation room during treatment as in the prior art. This improves the operability. In addition, this allows the operator to always clearly observe a picture of a morbid region without shifting his/her line of sight from the morbid region. This makes it possible to perform reliable surgical operations.

The magnetic sensor 10 detects the spatial moving amount of the head of the operator 38. The magnetic sensor 10 includes the magnetic sensor source unit 10b which generates a homogeneous magnetic field and the magnetic sensor sensing unit 10a which detects a magnetic field from the magnetic sensor source unit 10b. The magnetic sensor sensing unit 10a is mounted on an almost central portion of the HMD 9.

The magnetic sensor 10 with the above arrangement detects the movement of the head of the operator 38. More specifically, the magnetic sensor sensing unit 10a detects the homogenous magnetic field generated by the magnetic sensor source unit 10b set in a predetermined place other than the HMD 9 and processes information corresponding to a change in magnetic field accompanying the movement of the head to obtain the absolute spatial moving amount between the magnetic sensor source unit 10b and the magnetic sensor sensing unit 10a and an Euler angle (roll, pitch, and yaw) as the tilt of the magnetic sensor sensing unit 10a, thereby detecting the moving amount and inclination amount of the head of the surgeon.

The controller 11 includes a microcontroller 11a, DSPs 11b, servo drivers 11c, a magnetic sensor moving amount interface 11d, an up/down counter 11e, a keyboard interface unit 11f, a switch interface unit 11g, a hard disk controller unit 11h, a hard disk drive 11i, and data lines 11j, 11k, 11m, 11q, 11r, 11s, 11t, and 11u. The interactions between the respective modules of the controller 11 will be described below.

Data line 11m is a data bus line for sending position commands from the microcontroller 11a to the DSPs 11b, reading encoder feedback information of the servo unit on the slave arm side, inputting data from the up/down counter 11e, the magnetic sensor moving amount interface 11d, the keyboard interface unit 11f, and the switch interface unit 11g to the microcontroller 11a.

Data line 11j is an analog command line for sending the control computation results obtained by the DSPs 11b to the servo drivers 11c. Data lines 11k include a line for supplying power signals from the servo drivers 11c and a servo unit feedback encoder line.

Data line 11u is a data line for exchanging data between the hard disk drive 11i and the hard disk controller unit 11h. Data line 11t is a data line for exchanging data between the switch 12 and the switch interface unit 11g.

In this case, the information to be stored in the hard disk drive 11i includes, for example, teaching data for the observation slave manipulator 27 and treatment slave manipulators 25a and 25b, scale ratios, and various types of control parameters such as sensitivity. Obviously, it is possible to use, as a storage means, a storage medium used in information processing apparatus peripheral devices, for example, a CD, DVD, or external storage in place of the hard disk drive 11i. In addition, some kind of storage means such as external storage may be provided on the slave side.

Data line 11s is a data line for communication between the keyboard 13 and the keyboard interface unit 11f.

Although not shown, this system includes an address bus, control line, and the like for the selection of the respective functional modules. Although FIG. 1 shows only analog command lines as data lines 11j and 11k for driving the servo systems of the observation slave manipulator 27 and treatment slave manipulators 25a and 25b, the system also includes encoder feedback signal lines for making the DSPs 11b execute control algorithms such as PID control rules.

Using the above arrangement allows to perform the master-slave operation of making the observation slave manipulator 27 operate following (in accordance with) the movement of the head of the operator 38 (the movement of the HMD 9), and to perform the master-slave operation of making treatment slave manipulators 25a and 25b operate following (in accordance with) the manipulation of the master arm 8.

More specifically, treatment slave manipulators 25a and 25b perform master-slave operation in the following manner. The up/down counter 11e reads information from the encoder provided for the master arm 8 via data line 11q. The up/down counter 11e increases and decreases a moving amount with respect to the data set in the up/down counter 11e, and hence detects the absolute moving amount (the moving amount of the master arm 8). The microcontroller 11a receives the data held in the up/down counter 11e for each sampling operation via data line 11m.

The microcontroller 11a performs coordinate conversion processing for determining how to make the axes of treatment slave manipulators 25a and 25b operate with respect to the above moving amount.

In contrast, the observation slave manipulator 27 performs master-slave operation in the following manner. The HMD 9 sends information to the magnetic sensor moving amount interface 11d via data line 11r, thereby performing processing almost the same as the master-slave operation performed by treatment slave manipulators 25a and 25b.

As described above, a total of three slave manipulators, namely the observation slave manipulator 27, treatment slave manipulator 25a, and treatment slave manipulator 25b, are mounted on the bedside rails 3 of the operating table 1 on which the patient 2 is placed.

The endoscope 6 whose distal end is motor-driven to bend is connected to the distal end of the observation slave manipulator 27. The surgical instrument 4 whose distal end is motor-driven to bend and open/close is connected to the distal end of treatment slave manipulator 25a. The surgical instrument 4 whose distal end is motor-driven to bend and open/close is connected to the distal end of treatment slave manipulator 25b.

An actual arrangement example uses the two controllers 11-1 and 11-2 as the controller 11, as shown in FIG. 3.

Controller 11-1 performs control via a driving line 36a to make the operation of treatment slave manipulator 25a and observation slave manipulator 27 follow the operation of a master arm 8a manipulated by the operator 38 and of the HMD 9.

More specifically, controller 11-1 controls treatment slave manipulator 25a upon deciding the movement of the master arm 8 manipulated by the right hand of the operator, and controls the observation slave manipulator 27 upon deciding the movement of the head of the operator 38 via the HMD 9. More specifically, controller 11-1 receives the movement of the right hand of the operator 38 as information from the encoder provided for each joint of master arm 8a via a line 37a.

Controller 11-2 performs control via a driving line 36b to make the operation of treatment slave manipulator 25b follow the operation of a master arm 8b manipulated by the operator 38 and of the HMD 9.

More specifically, controller 11-2 controls treatment slave manipulator 25b upon deciding the movement of master arm 8b manipulated by the left hand of the operator. More specifically, controller 11-2 receives the movement of the left hand of the operator 38 as information from the encoder provided for each joint of master arm 8b via a line 37b.

The HMD 9 is mounted on the head of the operator 38. The magnetic sensor sensing unit 10a of the magnetic sensor is attached to the HMD 9. The magnetic sensor source unit 10b is disposed at a predetermined portion of master arm 8a. This arrangement detects the movement of the head of the operator 38 and sends the detected position information to controller 11-1 via a signal line 35.

The triple switch 12 constituted by the operation switch 12a, the emergency shutdown switch 12b, and the shutdown cancellation switch 12c is disposed near the operator 38 (operable range). The switch 12 is electrically connected to controller 11-1 via a line 33, and is also electrically connected to controller 11-2 via a line 34.

That is, on/off signals for the operation switch 12a and the emergency shutdown switch 12b are sent to controller 11-1 via the line 33. A processing circuit (not shown; corresponding to the microcontroller 11a shown in FIG. 1) in controller 11-1 then analyzes the signals. In addition, these signals are sent to controller 11-2 via the line 34. A processing circuit (not shown; corresponding to the microcontroller 11a shown in FIG. 1) in controller 11-2 then analyzes the signals. Controllers 11-1 and 11-2 then execute processing based on the analysis processing results.

When the operation switch 12a is turned on, controllers 11-1 and 11-2 perform control to allow master arms 8a and 8b to operate.

When the emergency shutdown switch 12b is turned on, this system forcibly shuts down the operation of a preset slave manipulator (to be described in detail later) of treatment slave manipulators 25a and 25b and the observation slave manipulator 27. In this case, the system shuts down controllers 11-1 and 11-2 based on predetermined determination results obtained by the microcontroller 11a.

When the shutdown cancellation switch 12c is turned on, the operation of treatment slave manipulators 25a and 25b and observation slave manipulator 27, which have been forcibly shut down when the emergency shutdown switch 12b or the emergency shutdown switch 24 (to be described later) was turned on, is resumed (made operable). In other words, the shutdown cancellation switch 12c is a means for canceling the shutdown state set by the emergency shutdown switch 12b or the emergency shutdown switch 24.

The shutdown cancellation switch 12c is not an indispensable constituent element, and the number of switches to be used may be one or more. Assume that the medical manipulator system does not include the shutdown cancellation switch 12c. In this case, after the system is forcibly shut down by the emergency shutdown switch 12b in the above manner, it is possible to restore the system by restarting it after its power supply is temporarily turned off.

The slave-side emergency shutdown switch 24 is a switch which is disposed on the slave side, i.e., inside the operable range of the assistant 138 near the operating table 1, as shown in FIG. 2, and has the same function as that of the emergency shutdown switch 12b.

As shown in FIG. 1, the slave-side emergency shutdown switch 24 is electrically connected to the switch interface unit 11g, as shown in FIG. 1. An on/off signal for the slave-side emergency shutdown switch 24 is input to controller 11-1 via the switch interface unit 11g. The processing circuit (not shown; corresponding to the microcontroller 11a shown in FIG. 1) in controller 11-1 then analyzes the signal. This signal is also sent to controller 11-2 via the line 34. The processing circuit (not shown) in controller 11-2 then analyzes the signal.

That is, when the slave-side emergency shutdown switch 24 is turned on, this system forcibly shuts down the operation of a preset slave manipulator of treatment slave manipulators 25a and 25b and the observation slave manipulator 27. In addition, the system turns off controllers 11-1 and 11-2 themselves depending on the predetermined determination result obtained by the microcontroller 11a.

The slave-side emergency shutdown switch 24 allows the assistant 138 stationed closer to the patient 2 than the operator 38 to perform emergency shutdown of treatment slave manipulators 25a and 25b and the observation slave manipulator 27 according to his/her own judgment.

Therefore, in case of emergency, for example, when treatment slave manipulator 25a or 25b or the observation slave manipulator 27 performs unexpected operation (for example, abrupt breakdown or runaway of a control program) or the condition of a patient has suddenly changed, it is possible to perform emergency shutdown of the corresponding manipulator more quickly and reliably. That is, this greatly improves the operability in case of an emergency.

When the shutdown cancellation switch 12c is turned on, the operation of treatment slave manipulators 25a and 25b and observation slave manipulator 27, which have been forcibly shut down when the emergency shutdown switch 24 was turned on, is resumed (operation shutdown state is cancelled).

Note that the emergency shutdown switch 12b and the slave-side emergency shutdown switch 24 may be formed as hand switches or foot switches. When, for example, the emergency shutdown switch 12b is provided as a hand switch, the switch may be provided on the operation grip portion of each of master arms 8a and 8b.

In addition, the range of shutdown by the emergency shutdown switch 12b and the slave-side emergency shutdown switch 24 may be commonly set for treatment slave manipulators 25a and 25b and the observation slave manipulator 27 or may be individually set for each slave manipulator. Individually setting this range for each slave manipulator will facilitate restoring operation.

More specifically, only a slave manipulator having great influence on a patient at the time of runaway may be set as an emergency shutdown target. That is, this system may have an arrangement in which a salve manipulator associated with a sharp surgical instrument is set as an emergency shutdown target, and a slave manipulator associated with a blunt surgical instrument, camera, or the like is not set as an emergency shutdown target. A surgical instrument is disposed to allow it to come into contact with a surgery site because of its purpose. In contrast, a camera can be disposed at a position where it does not come into contact with a surgery site even if the operator moves the camera in the entire movable range. For this reason, it is not always necessary to set the camera as an emergency shutdown target. This arrangement allows to keep providing visual information about a surgery target while executing emergency shutdown of a slave manipulator.

In addition, when a rigid scope is used as a camera, the slave manipulator associated with the camera is set as an emergency shutdown target. When a flexible scope is used, the camera may not be set as an emergency shutdown target in consideration of the fact that there is little influence on a patient because the flexible scope bends even if it comes into contact with an organ. This arrangement allows to smoothly continue a surgical operation even if a camera with a rigid scope is replaced with a camera with a flexible scope during the surgical operation.

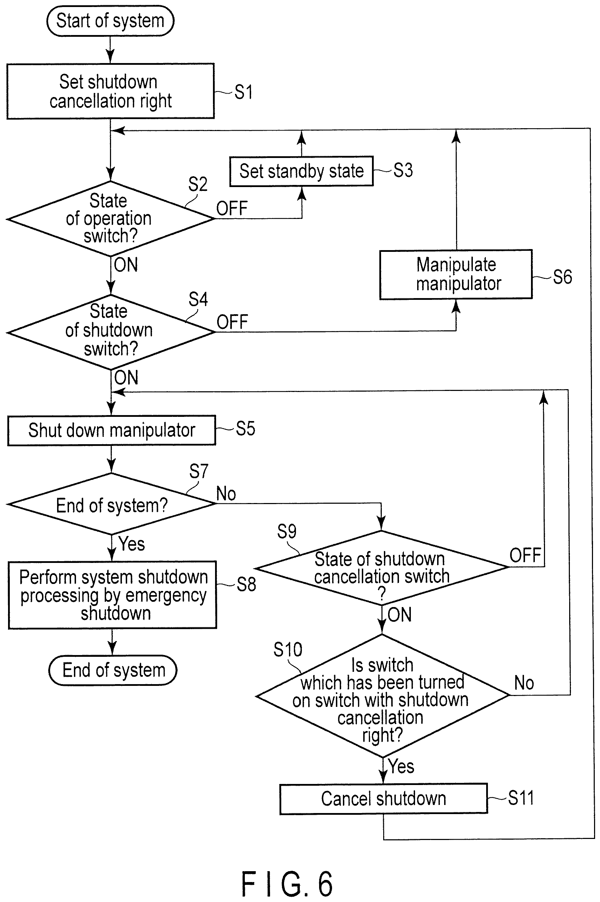

Processing unique to the medical manipulator system according to this embodiment (emergency shutdown processing and restoration processing by the microcontroller 11a) will be described in detail below with reference to the flowchart shown in FIG. 6.

For the sake of a clear description of the processing procedure, controllers 11-1 and 11-2 will be generically regarded as one controller 11 as shown in FIGS. 1 and 2. Note that the details of the processing performed by controllers 11-1 and 11-2 have already been described above.

First of all, the user of the medical manipulator system makes settings for "shutdown cancellation right" (step S1). This cancellation right is a right to restore the operation of treatment slave manipulators 25a and 25b and observation slave manipulator 27 which have undergone emergency shutdown by turning on the emergency shutdown switch 12b or the slave-side emergency shutdown switch 24.

As described above, a plurality of shutdown cancellation switches 12c are provided in some cases. In such a case, the user makes desired settings for shutdown cancellation rights for the shutdown cancellation switches 12c. More specifically, for example, the following setting methods are available:

### «Setting Example 1»

- A shutdown cancellation right is set for only the specific shutdown cancellation switch 12c.

More specifically, if there are a plurality of operators 38, a shutdown cancellation right is set for only the shutdown cancellation switch 12c corresponding to the main operator 38. Alternatively, a shutdown cancellation right is set for only the shutdown cancellation switch 12c corresponding to the operator 38 closest to the operating table 1. Alternatively, this system may be configured to set a shutdown cancellation right for only the operator 38 or the assistant 138.

### «Setting Example 2»

- Shutdown cancellation rights are set for all the shutdown cancellation switches 12c, and settings are made to execute shutdown cancellation processing when any of the shutdown cancellation switches 12c is turned on.

### «Setting Example 3»

- Shutdown cancellation rights are set for all the shutdown cancellation switches 12c, and settings are made to execute shutdown cancellation processing only when all the shutdown cancellation switches 12c are turned on.

Introducing shutdown cancellation rights in the above manner can properly restore treatment slave manipulators 25a and 25b and the observation slave manipulator 27 which have undergone emergency shutdown.

For example, the relationship between the operator 38 and the assistant 138 varies for each surgical operation. In some surgical operation, for example, an experienced doctor serves as an assistant, and a doctor who is accumulating experience serves as an operator. In such a case, the experienced doctor can make a more accurate decision more quickly. In this case, therefore, it is preferable to set a shutdown cancellation right for the slave-side emergency shutdown switch 24 near the assistant (near the patient 2) (set a switch (not shown) corresponding to the shutdown cancellation switch 12c near the slave-side emergency shutdown switch 24).

Specific setting methods for shutdown cancellation rights include a method of making settings in a hardware manner and a method of making settings in a software manner. The user may use any of these methods.

Obviously, the user may set a shutdown cancellation right permanently instead of setting a shutdown cancellation right in step S1 in each case.

Upon completing shutdown cancellation right setting processing in step S1, the system makes treatment slave manipulators 25a and 25b and the observation slave manipulator 27 ready for operation.

Based on the state of the operation switch 12a, the microcontroller 11a then determines whether to start operation or maintain the operation ready state (standby state) (step S2). The system maintains the operation ready state (standby state) until determining in step S2 that the operation switch 12a is in the on-state (step S3).

In addition, the microcontroller 11a determines the on/off states of the emergency shutdown switch 12b and slave-side emergency shutdown switch 24 (step S4).

Upon determining in step S4 that at least one of the emergency shutdown switch 12b and the slave-side emergency shutdown switch 24 is in the on-state, the microcontroller 11a forcibly shuts down the operation of treatment slave manipulators 25a and 25b and observation slave manipulator 27 (step S5). In contrast, upon determining in step S4 that both the emergency shutdown switch 12b and the slave-side emergency shutdown switch 24 are in the off-state, the system makes each slave manipulator operate (step S6).

That is, if the operation switch 12a is in the on-state and the emergency shutdown switch 12b and the slave-side emergency shutdown switch 24 are in the off-state, the system continuously repeats the processing in step S6, continues the operation of the manipulator, and executes treatment and observation.

That is, step S4 is a step of detecting the on/off states of the emergency shutdown switch 12b and the slave-side emergency shutdown switch 24 and makes the process branch based on the detection result.

Upon forcibly shutting down treatment slave manipulators 25a and 25b and the observation slave manipulator 27 in step S5, the microcontroller 11a determines whether to shut down the controller 11 (step S7).

More specifically, in step S7, the process may shift to the step of waiting for an input instruction whether to shut down the system and performing decision. More specifically, there are available a method of providing a dedicated switch (not shown), a method of shutting down the system by pressing the emergency shutdown switch 12b or the slave-side emergency shutdown switch 24 again, a method of shutting down the system unless restoring operation is performed in a predetermined period of time, and the like.

If step S7 branches to "YES", the microcontroller 11a shuts down the controller 11 and shuts down the medical manipulator system (step S8).

In step S8, in order to perform shutdown by forcible shutdown in case of an emergency, information indicating that "this is not shutdown by normal termination" may be recorded in a predetermined memory. More specifically, for example, information indicating "By which emergency shutdown switch is operated to carried out the shutdown" or the like is recorded in the memory.

Note that shutdown by normal termination is irrelevant to the gist of the present invention, and hence a description of it will be omitted. Likewise, a description of the details of initialization processing at the startup of the system will be omitted.

If the system is shut down by the processing in step S8, at the time of next starting the system may present the user with information indicating that "the previous termination processing was not normal termination processing".

If step S7 branches to "NO", the microcontroller 11a determines the on/off state of the shutdown cancellation switch 12c (step S9). If the microcontroller 11a determines in step S9 that the shutdown cancellation switch 12c is in the off-state, the process returns to step S5.

If the microcontroller 11a determines in step S9 that the shutdown cancellation switch 12c is in the on-state, the microcontroller 11a determines whether the above shutdown cancellation right is set for the shutdown cancellation switch 12c (step S10). If step S10 branches to "NO", the process returns to step S5. If step S10 branches to "YES", the microcontroller 11a performs the processing of resuming the operation of treatment slave manipulators 25a and 25b and observation slave manipulator 27 which have been forcibly shut down (step S11). The process then returns to step S2.

More specifically, in step S11, for example, the system prompts the operator to turn off the operation switch 12a or forcibly turns off the operation switch 12a. This makes it possible to start the operation of each slave manipulator only when the operation switch 12a is turned on, after the shutdown is cancelled, in the same manner as that at the time of startup, instead of starting the operation immediately after the shutdown is cancelled. This can reduce the possibility of erroneous operation.

Note that the processing in step S5 is mechanical power shutdown processing (operation shutdown processing for a slave manipulator with a clutch), and the processing in step S8 is electrical power shutdown processing. Performing forcible shutdown of the operation of a slave manipulator in two steps can facilitate restoration after the forcible shutdown is cancelled, and can realize proper, reliable forcible shutdown.

More specifically, using mechanical power shutdown processing implemented by a clutch in a hardware manner as the clutch function for slave manipulator driving in step S5 can reliably shut down operation even at the time of runaway of software. For example, the system is provided with an electromagnetic clutch of an excitation actuation type to perform mechanical power shutdown by releasing a solenoid-driven electromagnetic clutch by shutting down power supply to the clutch. In addition, using mechanical power shutdown processing implemented by a software clutch in step S5 allows to make flexible settings in the processing. For example, it is possible to shut down operation by a method of making the microcontroller 11a stop issuing a command, a method of switching the servo driver 11c to the disable state, or the like.

As described above, this embodiment can provide a medical manipulator system with improved operability on the slave side.

More specifically, the medical manipulator system according to this embodiment solves the following problems in the prior art.

That is, the operator of a medical manipulator system closely observes an image of a surgery site in a patient which is displayed on a monitor, and hence may not quickly notice an abnormal situation such as interference between slave manipulators outside the body of the patient or a sudden change in the condition of the patient. In consideration of such situations, a technique like that disclosed in Japanese Patent No. 3717552 has been proposed. Even use of this technique allows only the operator to perform emergency shutdown. Therefore, an assistant needs to inform the operator of the current situation to make the operator perform emergency shutdown. Obviously, in this case, there is a time loss due to information transfer from the assistant to the operator. In addition, the operator needs to perform quick operation. This increases the possibility of erroneous operation.

With regard to the above problems, according to the medical manipulator system of this embodiment, the slave-side emergency shutdown switch 24 allows the assistant 138 closer to the patient 2 than the operator 38 to perform emergency shutdown of treatment slave manipulators 25a and 25b and the observation slave manipulator 27 according to his/her own judgment.

It is therefore possible to shut down treatment slave manipulators 25a and 25b and the observation slave manipulator 27 more quickly and reliably in case of an emergency at the time of, for example, abnormal operation of the manipulators. Since the assistant 138 moves during a surgical operation, a plurality of emergency shutdown switches may be disposed. For example, an emergency shutdown switch may be disposed for each manipulator.

Note that an emergency shutdown display means (for example, an LED) which blinks in red in case of an emergency may be disposed at a position where the operator 38 can easily see (for example, a position near master arm 8a or 8b or a position near the controller 11).

In an actual surgical field, the operator of a medical manipulator system of such a master-slave type often touches an unsterilized input device because it is difficult to sterilize it. That is, in an actual surgical field, an operator sometimes performs operation from a so-called unclean area.

On the other hand, an assistant who is stationed near a patient to replace surgical tools and support treatment needs to directly touch a surgery site and the patient, and hence sterilizes his/her hands or wears sterilized gloves. That is, the assistant supports treatment from a so-called clean area. In this manner, in an actual surgical field, an operator and an assistant separately perform a surgical operation from an unclean area and a clean area, respectively. If, therefore, an unclean area and a clean area cross each other, the patient may develop an infection.

The medical manipulator system according to this embodiment, however, is configured to prevent an unclean area and a clean area from crossing each other (is configured to allow emergency shutdown operation/shutdown cancellation operation in both the unclean area and the clean area), and hence is free from the above problem.

If this system is formed by using hand switches, hand switches disposed in a clean area may be sterilized or covered with a sterile cover (drape). Hand switches are superior to foot switches in that, unlike foot switches, they offer good visibility and operability, avoid the possibility of an assistant tripping over a wire when he/she moves, and avoid the possibility that the assistant accidentally steps on one of them.

In addition, the system may be configured to use a combination of a plurality of types of switches, for example, a foot switch as the operator-side emergency shutdown switch 12b and a hand switch as the slave-side emergency shutdown switch 24.

Obviously, in some cases, both an input device and a manipulator are installed in a clean area. Even in such a case, disposing an emergency shutdown switch at a position where an assistant can operate will have the same merit (effect) as that described above.

As described above, according to the medical manipulator system of this embodiment, in a surgical operation using a plurality of medical manipulators, even if some kind of abnormal operation or operation failure occurs in any of the manipulators, it is possible to quickly and accurately respond to such a situation. This can achieve excellent operability and reliability, shorten the time required for a surgical operation, and reduce invasiveness for the patient.

Although the present invention has been described with reference to one embodiment, the present invention is not limited to the embodiment described above and can be modified and applied without departing from the essence of the present invention. In addition, although the medical manipulator of the master-slave scheme has been described above, this embodiment can be applied to manipulators other than that of the master-slave scheme, for example, a manipulator having no coordinate conversion processing between operation input and manipulator operation and a medical manipulator which operates along a pre-installed program.

### [Modification]

A modification of the medical manipulator system according to this embodiment will be described below. In order to avoid a redundant description, differences from the medical manipulator according to the embodiment will be described.

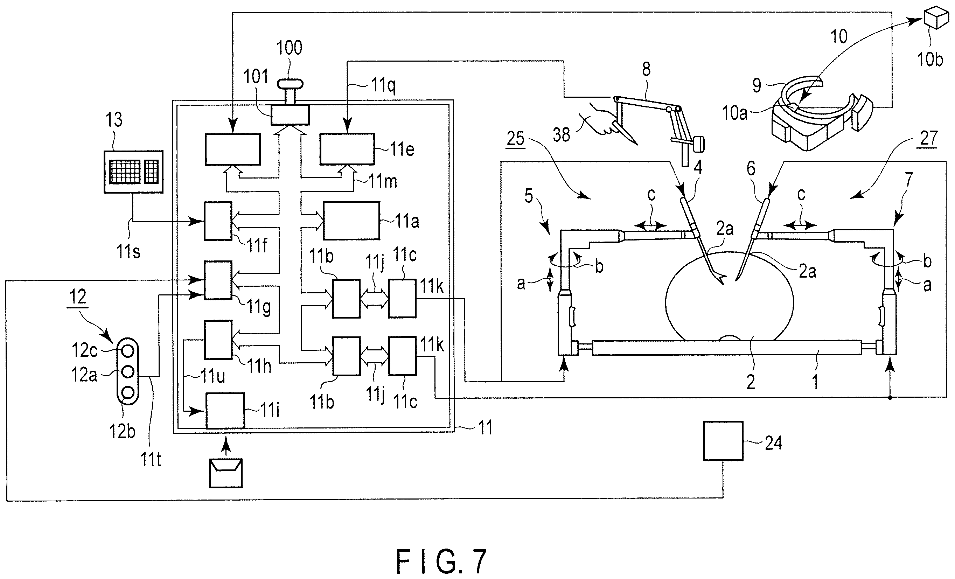

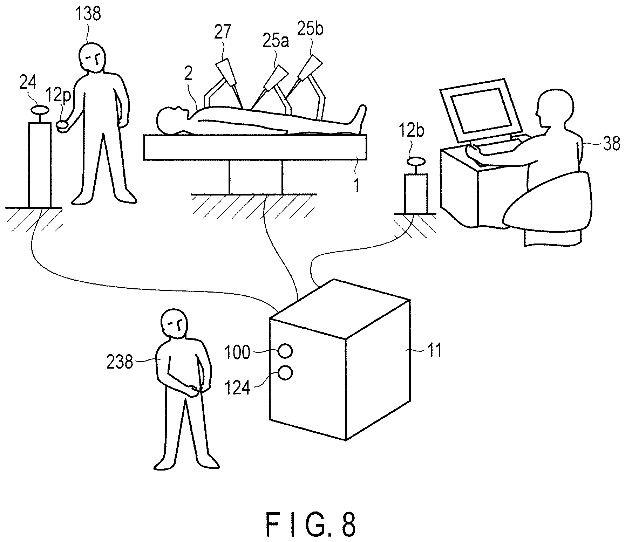

FIG. 7 is a view showing an example of the arrangement of a medical manipulator system according to a modification of the above embodiment. FIG. 8 is a schematic view showing an actual image of the medical manipulator system according to the modification of the embodiment.

As shown in FIGS. 7 and 8, the medical manipulator system according to this embodiment is further provided with a portable emergency shutdown switch 12p as an emergency shutdown switch having the same function as that of the emergency shutdown switch 12b and slave-side emergency shutdown switch 24. The controller 11 is provided with a communication unit 100 for communication with the portable emergency shutdown switch 12p and an I/O port 101.

The portable emergency shutdown switch 12p is an emergency shutdown switch which is portable and is held by, for example, the assistant 138 or the like on the slave side.

A signal representing the on/off state of the portable emergency shutdown switch 12p is transmitted to the communication unit 100 wirelessly or by wire. The signal transmitted from the portable emergency shutdown switch 12p is received by the communication unit 100 of the controller 11 and input to the microcontroller 11a via the I/O port 101.

As has been described above, this modification can provide a medical manipulator system which has the same effects as those of the medical manipulator system according to this embodiment and also has, for example, the following effect.

Assume that the assistant 138 is stationed in a place remote from the slave-side emergency shutdown switch 24 when he/she must perform emergency shutdown of treatment slave manipulators 25a and 25b and the observation slave manipulator 27 at the occurrence of an abnormal event. Even in this case, the assistant 138 can quickly perform emergency shutdown processing. That is, this can further improve the operability and reliability.

In order to further improve the operability and reliability, the controller 11 may be provided with a controller-side emergency shutdown switch 124, as shown in FIG. 8. This arrangement allows a medical engineer (ME) 238 to perform the above emergency shutdown processing according to his/her judgment, if he/she is stationed near the controller 11. The ME is most knowledgeable about the technical matter (control programs and the like) of the controller 11, and hence can quickly notice such abnormalities and perform shutdown operation.

The above embodiments include inventions of various stages, and various inventions can be extracted by proper combinations of a plurality of disclosed constituent elements. When, for example, the problems described in "Technical Problem" can be solved and at least one of the effects described in "Advantageous Effects of Invention" can be obtained even if several constituent elements are omitted from all the constituent elements in each embodiment, the arrangement from which these constituent elements are omitted can be extracted as an invention.

## Claims

1. A medical manipulator system controlled by an operator (38) to perform diagnosis or treatment of a living body of a patient (2), the medical manipulator system comprising:
an operating table (1) for placing the patient (2);
a single or a plurality of manipulators (25, 25a, 25b, 27) configured to hold a medical tool;
a control unit (11) configured to control operation of the manipulator or manipulators;
a first forcible shutdown instruction unit (24, 12p) disposed within an operable range of an assistant (138) located within an operable range of the operating table (1); and **characterized in that** the system further comprises
a second forcible shutdown instruction unit (12) disposed within an operable range of the operator (38) disposed remote from the operating table (1) or in a different room from the operating table (1),
wherein the first and second forcible shutdown instructions units (12b, 24, 12p) are configured to instruct the control unit (11) to forcibly shut down operation of the manipulator or manipulators (25, 25a, 25b, 27), and
wherein, the control unit (11) forcibly shuts down operation of the single manipulator, of a predetermined manipulator of the plurality of manipulators (25, 25a, 25b, 27) or of all manipulators of the plurality of manipulators (25, 25a, 25b, 27) based on an instruction from at least one of the first and second forcible shutdown instruction units (12b,24,12p) and then the control unit (11) determines to shut down the medical manipulator system if a further instruction from the same forcible shutdown instruction unit (24,12p,12b) is performed in a predetermined period of time.

2. The medical manipulator system of claim 1, wherein the first forcible shutdown instruction unit (24, 12p) comprises a plurality of forcible shutdown instruction units.

3. The medical manipulator system of claim 1, further comprising an operation restart instruction unit (12a) configured to restart operation of the single or predetermined manipulator or the plurality of manipulators (25, 25a, 25b, 27), which has been forcibly shut down by the control unit (11) based on an instruction from the forcible shutdown instruction unit (12, 24, 12p).

4. The medical manipulator system of claim 3, wherein the operation restart instruction unit (12a) comprises a plurality of operation restart instruction units (12a), and a right to execute the operation restart instruction is set for at least one of the operation restart instruction units (12a).

5. The medical manipulator system of claim 4, wherein the right to execute the operation restart instruction is set for a specific one of the operation restart instruction units (12a).

6. The medical manipulator system of claim 4, wherein rights to execute the operation restart instruction are set for all the operation restart instruction units (12a).

7. The medical manipulator system of claim 4, wherein when all the operation restart instruction units (12a) issue operation restart instructions, the operation restart instruction is executed.

8. The medical manipulator system of claim 1, wherein the forcible shutdown instruction unit (12, 24, 12p) is configured to move, and to communicate with the control unit (11) wirelessly and/or by wire.

9. The medical manipulator system of claim 1, wherein the forcible shutdown instruction unit (12, 24, 12p) is set to set only a predetermined manipulator (25, 25a, 25b, 27) of the plurality of manipulators (25, 25a, 25b, 27) as a target for the forcible shutdown.

10. The medical manipulator system of any one of claims 1 to 9, wherein the manipulator system comprises a manipulator (25, 25a, 25b, 27) of a master-slave scheme.

## Patentansprüche

1. Medizinisches Manipulatorsystem, das von einem Bediener (38) gesteuert wird, um eine Diagnose oder Behandlung eines lebenden Körpers eines Patienten (2) durchzuführen, wobei das medizinische Manipulatorsystem umfasst:
einen Operationstisch (1) zum Platzieren des Patienten (2);
einen einzigen oder mehrere Manipulatoren (25, 25a, 25b, 27), der bzw. die dazu konfiguriert ist bzw. sind, ein medizinisches Instrument zu halten;
eine Steuereinheit (11), die dazu konfiguriert ist, eine Bedienung des Manipulators oder der Manipulatoren zu steuern;
eine erste Einheit (24, 12p) zur Anweisung einer erzwungenen Abschaltung, die innerhalb eines Bedienungsbereichs eines Assistenten (138) angeordnet ist, der sich innerhalb eines Bedienungsbereichs des Operationstischs (1) befindet; und **dadurch gekennzeichnet, dass** das System weiterhin umfasst:
eine zweite Einheit (12) zur Anweisung einer erzwungenen Abschaltung, die innerhalb eines Bedienungsbereichs des Bedieners (38) angeordnet ist, der sich entfernt von dem Operationstisch (1) oder in einem anderen Raum von dem Operationstisch (1) befindet,
wobei die erste und die zweite Einheit (12b, 24, 12p) zur Anweisung einer erzwungenen Abschaltung dazu konfiguriert sind, die Steuereinheit (11) anzuweisen, eine Bedienung des Manipulators oder der Manipulatoren (25, 25a, 25b, 27) auf erzwungene Weise abzuschalten, und
wobei die Steuereinheit (11) die Bedienung des einzigen Manipulators, eines vorherbestimmten Manipulators der mehreren Manipulatoren (25, 25a, 25b, 27) oder aller Manipulatoren der mehreren Manipulatoren (25, 25a, 25b, 27) auf der Basis einer Anweisung von mindestens einer der ersten und der zweiten Einheit (12b, 24, 12p) zur Anweisung einer erzwungenen Abschaltung auf erzwungene Weise abschaltet und die Steuereinheit (11) dann bestimmt, das medizinische Manipulatorsystem abzuschalten, wenn eine weitere Anweisung von derselben Einheit (24, 12p, 12b) zur Anweisung einer erzwungenen Abschaltung in einem vorherbestimmten Zeitraum durchgeführt wird.

2. Medizinisches Manipulatorsystem nach Anspruch 1, wobei die erste Einheit (24, 12p) zur Anweisung einer erzwungenen Abschaltung mehrere Einheiten zur Anweisung einer erzwungenen Abschaltung umfasst.

3. Medizinisches Manipulatorsystem nach Anspruch 1, weiterhin umfassend eine Bedienungsneustartanweisungseinheit (12a), die dazu konfiguriert ist, eine Bedienung des einzigen oder vorherbestimmten Manipulators oder der mehreren Manipulatoren (25, 25a, 25b, 27) neu zu starten, die von der Steuereinheit (11) auf der Basis einer Anweisung von der Einheit (12, 24, 12p) zur Anweisung einer erzwungenen Abschaltung auf erzwungene Weise abgeschaltet wurde.

4. Medizinisches Manipulatorsystem nach Anspruch 3, wobei die Bedienungsneustartanweisungseinheit (12a) mehrere Bedienungsneustartanweisungseinheiten (12a) umfasst und ein Recht zum Ausführen der Bedienungsneustartanweisung für mindestens eine der Bedienungsneustartanweisungseinheiten (12a) eingestellt ist.

5. Medizinisches Manipulatorsystem nach Anspruch 4, wobei das Recht zum Ausführen der Bedienungsneustartanweisung für eine spezifische der Bedienungsneustartanweisungseinheiten (12a) eingestellt ist.

6. Medizinisches Manipulatorsystem nach Anspruch 4, wobei Rechte zum Ausführen der Bedienungsneustartanweisung für alle der Bedienungsneustartanweisungseinheiten (12a) eingestellt sind.

7. Medizinisches Manipulatorsystem nach Anspruch 4, wobei, wenn alle der Bedienungsneustartanweisungseinheiten (12a) Bedienungsneustartanweisungen erteilen, die Bedienungsneustartanweisung ausgeführt wird.

8. Medizinisches Manipulatorsystem nach Anspruch 1, wobei die Einheit (12, 24, 12p) zur Anweisung einer erzwungenen Abschaltung dazu konfiguriert ist, die Steuereinheit (11) zu bewegen und mit dieser drahtlos und/oder drahtgebunden zu kommunizieren.

9. Medizinisches Manipulatorsystem nach Anspruch 1, wobei die Einheit (12, 24, 12p) zur Anweisung einer erzwungenen Abschaltung dazu eingestellt ist, nur einen vorherbestimmten Manipulator (25, 25a, 25b, 27) der mehreren Manipulatoren (25, 25a, 25b, 27) als ein Ziel für die erzwungene Abschaltung einzustellen.

10. Medizinisches-Manipulatorsystem-nach-einem-der Ansprüche 1 bis 9, wobei das Manipulatorsystem einen Manipulator (25, 25a, 25b, 27) eines Master-Slave-Schemas umfasst.

## Revendications

1. Système de manipulation médicale commandé par un opérateur (38) pour réaliser un diagnostic ou un traitement d'un corps vivant d'un patient (2), le système de manipulation médicale comprenant :
une table d'opération (1) pour placer le patient (2) ;
un unique manipulateur ou une pluralité de manipulateurs (25, 25a, 25b, 27) configurés pour tenir un outil médical ;
une unité de commande (11) configurée pour commander le fonctionnement du ou des manipulateurs ;
une première unité d'instruction d'arrêt forcé (24, 12p) disposée dans les limites d'une portée opérationnelle d'un assistant (138) situé dans les limites d'une portée opérationnelle de la table d'opération (1) ; et **caractérisé par le fait que** le système comprend en outre :
une seconde unité d'instruction d'arrêt forcé (12) disposée dans les limites d'une portée opérationnelle de l'opérateur (38) situé à distance de la table d'opération (1) ou dans une salle différente de la table d'opération (1),
les première et seconde unités d'instruction d'arrêt forcé (12b, 24, 12p) étant configurées pour ordonner à l'unité de commande (11) d'arrêter de manière forcée le fonctionnement du manipulateur ou des manipulateurs (25, 25a, 25b, 27), et
l'unité de commande (11) arrêtant de manière forcée le fonctionnement de l'unique manipulateur, d'un manipulateur prédéterminé parmi la pluralité de manipulateurs (25, 25a, 25b, 27) ou de tous les manipulateurs parmi la pluralité de manipulateurs (25, 25a, 25b, 27) sur la base d'une instruction provenant d'au moins une parmi les première et seconde unités d'instruction d'arrêt forcé (12b, 24, 12p), puis l'unité de commande (11) déterminant d'arrêter le système de manipulation médicale si une autre instruction provenant de la même unité d'instruction d'arrêt forcé (24, 12p, 12b) est réalisée pendant une période de temps prédéterminée.

2. Système de manipulation médicale selon la revendication 1, dans lequel la première unité d'instruction d'arrêt forcé (24, 12p) comprend une pluralité d'unités d'instruction d'arrêt forcé.

3. Système de manipulation médicale selon la revendication 1, comprenant en outre une unité d'instruction de redémarrage de fonctionnement (12a) configurée pour redémarrer le fonctionnement de l'unique manipulateur ou du manipulateur prédéterminé ou de la pluralité de manipulateurs (25, 25a, 25b, 27), qui a été arrêté de manière forcée par l'unité de commande (11) sur la base d'une instruction provenant de l'unité d'instruction d'arrêt forcé (12, 24, 12p).

4. Système de manipulation médicale selon la revendication 3, dans lequel l'unité d'instruction de redémarrage de fonctionnement (12a) comprend une pluralité d'unités d'instruction de redémarrage de fonctionnement (12a), et un droit d'exécuter l'instruction de redémarrage de fonctionnement est défini pour au moins une des unités d'instruction de redémarrage de fonctionnement (12a).

5. Système de manipulation médicale selon la revendication 4, dans lequel le droit d'exécuter l'instruction de redémarrage de fonctionnement est défini pour une unité spécifique parmi les unités d'instruction de redémarrage de fonctionnement (12a).

6. Système de manipulation médicale selon la revendication 4, dans lequel des droits d'exécuter l'instruction de redémarrage de fonctionnement sont définis pour toutes les unités d'instruction de redémarrage de fonctionnement (12a).

7. Système de manipulation médicale selon la revendication 4, dans lequel, lorsque toutes les unités d'instruction de redémarrage de fonctionnement (12a) émettent des instructions de redémarrage de fonctionnement, l'instruction de redémarrage de fonctionnement est exécutée.

8. Système de manipulation médicale selon la revendication 1, dans lequel l'unité d'instruction d'arrêt forcé (12, 24, 12p) est configurée pour se déplacer et communiquer avec l'unité de commande (11) de manière sans fil et/ou filaire.

9. Système de manipulation médicale selon la revendication 1, dans lequel l'unité d'instruction d'arrêt forcé (12, 24, 12p) est définie pour définir uniquement un manipulateur prédéterminé (25, 25a, 25b, 27) parmi la pluralité de manipulateurs (25, 25a, 25b, 27) comme cible pour l'arrêt forcé.

10. Système de manipulation médicale selon l'une quelconque des revendications 1 à 9, le système de manipulation comprenant un manipulateur (25, 25a, 25b, 27) d'un schéma maître-esclave.
